# EUROPEAN PATENT SPECIFICATION

(11) **EP 4 380 439 B1**
(45) Date of publication and mention of the grant of the patent: **04.11.2026**
(21) Application number: 22778151.5
(22) Date of filing: 07.09.2022
(51) Int. Cl.: A61B 5/024, A61B 5/00, A61M 21/00

(54) **CONFIGURABLE WAKE UP ALARM USING PHYSIOLOGICAL MONITORING**
KONFIGURIERBARER AUFWECKALARM MIT PHYSIOLOGISCHER ÜBERWACHUNG
ALARME DE RÉVEIL CONFIGURABLE UTILISANT LA SURVEILLANCE PHYSIOLOGIQUE

(30) Priority: 07.09.2021 US 202163241282 P; 13.10.2021 US 202163255043 P
(43) Date of publication of application: 12.06.2024
(73) Proprietor: Whoop, Inc., Boston, MA 02215 (US)
(72) Inventor: CAPODILUPO, Emily, Rachel, Boston, MA 02215 (US); CAPODILUPO, John, Vincenzo, Boston, MA 02215 (US); GRACE, Julia, Susan, Boston, MA 02215 (US); GREENE, Mark, Jonathan, Boston, MA 02215 (US); WAY, Marcus, Nye, Boston, MA 02215 (US)
(74) Representative: Black, Diego
(86) International application number: PCT/US2022/042682
(87) International publication number: WO 2023/038919

(56) References cited:
- WO-A2-2015/006364
- US-A1- 2015 164 238
- US-A1- 2017 209 055

## Description

### CROSS-REFERENCE TO RELATED APPLICATIONS

This application claims priority to U.S. Provisional Patent App. No. 63/241,282 filed on September 7, 2021 and U.S. Provisional Patent App. No. 63/255,043 filed on October 13, 2021.

### BACKGROUND

There remains a need for improved alarm systems that wake users based on physiological data.
US2017209055 discloses devices and methods for non-invasively measuring arterial stiffness using pulse wave analysis of photoplethysmogram data. In some implementations, wearable biometric monitoring devices provided herein for measuring arterial stiffness have the ability to automatically and intelligently obtain PPG data under suitable conditions while the user is engaged in activities or exercises. WO2015006364 discloses a processing system includes methods to promote sleep. The system may include a monitor such as a non-contact motion sensor from which sleep information may be determined. User sleep information, such as sleep stages, hypnograms, sleep scores, mind recharge scores and body scores, may be recorded, evaluated and/or displayed for a user. US2015164238 discloses a sleep systems having embedded sensors are described. In one aspect, a sleep system includes a mattress and one or more force sensors embedded within the mattress. The force sensors are positioned within the mattress to sense movement of an occupant of the mattress.

### SUMMARY

The invention is defined by the appended claims.

A controllable window of time is provided for waking a user from sleep. A system uses this window to variably control the acquisition of physiological data from a device such as a wearable monitor, such as by initiating data acquisition at the beginning of the window, and the acquired data can be used in turn to control when, during the window, an active alarm to the user might be provided. Using this technique, data acquisition from a physiological monitoring device or the like can be increased around the onset of the window to more accurately calculate a suitable waking time for the user within the window. This advantageously avoids the need for continuous, high-frequency data communications during long intervals of sleep, and focuses data transmission, related communications, and computing resources on those intervals when up-to-date data might be most useful for optimizing the user's wake up experience.

In an aspect, a computer program product disclosed herein may include computer executable code embodied in a non-transitory computer readable medium that, when executing on one or more computing devices, performs the steps of: storing a window on a wearable heart rate monitor, the window configured by a user for waking the user from a sleep event and the window timewise bounded by an onset of a waking interval and an end of the waking interval; monitoring a heart rate signal with the wearable heart rate monitor to acquire heart rate data; periodically transmitting the heart rate data through a smart phone of the user to a remote server during the sleep event at a first frequency; at the onset of the window, transmitting the heart rate data to the remote server at a second frequency greater than the first frequency; processing the heart rate data at the remote server to determine whether a wake signal should be issued from the remote server to awaken the user before the end of the window; if the wake signal is received from the remote server during the window, generating a haptic output with the wearable heart rate monitor to wake the user at a wake time within the window specified by the wake signal; and, if the wake signal is not received from the remote server during the window, generating the haptic output to wake the user at the end of the window.

In an aspect, a method disclosed herein may include: storing a window on a wireless monitoring device, the window configured by a user for waking the user from a sleep event and the window timewise bounded by an onset and an end; monitoring a physiological signal associated with sleep quality with the wireless monitoring device to acquire physiological data; at the onset of the window, altering an attribute related to communication of the physiological data from the wireless monitoring device to a remote processing system; if a wake signal is received from the remote processing system during the window, generating an output to wake the user at a wake time within the window specified by the wake signal; and, if the wake signal is not received from the remote processing system during the window, generating the output to wake the user at the end of the window.

Implementations may include one or more of the following features. Altering the attribute may include an increase of one or more of a frequency, a resolution, and a data rate of the physiological data. Altering the attribute may include an adjustment of a data type. The user may provide the end to the window as a time when the user must wake up. The user may provide the onset to the window as an earliest time when the user wishes to wake up. The onset of the window may be automatically calculated in response to a selection by the user of the end of the window. The end of the window may be automatically calculated in response to a selection by the user of the onset of the window. At least one of the onset and the end of the window may be automatically calculated for the user. At least one of the onset and the end of the window may be calculated for the user based on a sleep need of the user. At least one of the onset and the end of the window may be calculated for the user based on a prior day strain for the user. The remote processing system may evaluate sleep of the user based on the physiological data and may determine whether to issue the wake signal based on a quality of the sleep. The remote processing system may evaluate sleep of the user based on the physiological data and may determine whether to issue the wake signal based on a stage of the sleep. The wireless monitoring device may include a wrist-worn physiological monitor. The wireless monitoring device may include a photoplethysmography device. The remote processing system may include a smart phone associated with the user and the wireless monitoring device. The remote processing system may include a remote server configured to analyze sleep performance based on the physiological signal. The output may include a haptic device or an audio device on the wireless monitoring device. The output may include an audio device on a smart phone associated with the user. The wake signal may include a timestamp indicating a wake up time calculated by the remote processing system. The wake signal may not include a timestamp indicating a wake up time calculated by the remote processing system.

In an aspect, a system disclosed herein may include a wearable physiological monitoring device including a memory storing a window timewise bounded by an onset and an end configured by a user for waking the user from a sleep event, the wearable physiological monitoring device further including a haptic output device and a first wireless interface. The system may also include a personal electronic device associated with the user, the personal electronic device providing an interface for the user to configure the window and the personal electronic device including a second wireless interface coupled in a communicating relationship with the first wireless interface of the wearable physiological monitoring device. The system may also include a remote processing resource coupled through a data network to the personal electronic device, the remote processing resource configured to receive physiological data acquire by the wearable physiological monitoring device and communicated to the remote processing resource through the personal electronic device, the remote processing resource further configured to analyze the physiological data and to conditionally issue a wake signal to the wearable physiological monitoring device prior to the end of the window when an analysis of the physiological data shows an optimum time to wake the user before the end of the window. The wearable physiological monitoring device may be responsive to a receipt of the wake signal from the remote processing resource by outputting a signal to the haptic output device to wake the user.

In an aspect, a method disclosed herein may include: acquiring physiological data with a wearable monitoring device of a user; storing the physiological data in a memory of the wearable monitoring device; batch transferring the physiological data to a remote processing resource for evaluation of a wake time for the user at a beginning of a window for waking the user; continuously transmitting additional physiological data to the remote processing resource during the window; and generating a waking alarm for the user at an earliest of an expiration of the window or a receipt of a wake signal from the remote processing resource.

### BRIEF DESCRIPTION OF THE DRAWINGS

The foregoing and other objects, features, and advantages of the devices, systems, and methods described herein will be apparent from the following description of particular embodiments thereof, as illustrated in the accompanying drawings. The drawings are not necessarily to scale, emphasis instead being placed upon illustrating the principles of the devices, systems, and methods described herein. In the drawings, like reference numerals generally identify corresponding elements.
Fig. 1 shows a device for wearable physiological monitoring.
Fig. 2 is a block diagram of a computing device that may be used herein.
Fig. 3 illustrates an environmental and physiological monitoring system.
Fig. 4 is a flow chart of a method for providing a configurable wake up alarm.
Fig. 5 is a flow chart of a method for generating a waking alarm.
Fig. 6 is a flow chart of a method for generating a waking alarm.

### DETAILED DESCRIPTION

Embodiments will now be described more fully hereinafter with reference to the accompanying figures, in which preferred embodiments are shown. The foregoing may, however, be embodied in many different forms and should not be construed as limited to the illustrated embodiments set forth herein. Rather, these illustrated embodiments are provided so that this disclosure will convey the scope to those skilled in the art.

References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth.

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately" or the like, when accompanying a numerical value, are to be construed as indicating a deviation as would be appreciated by one of ordinary skill in the art to operate satisfactorily for an intended purpose. Similarly, words of approximation such as "approximately" or "substantially" when used in reference to physical characteristics, should be understood to contemplate a range of deviations that would be appreciated by one of ordinary skill in the art to operate satisfactorily for a corresponding use, function, purpose, or the like. Ranges of values and/or numeric values are provided herein as examples only, and do not constitute a limitation on the scope of the described embodiments. Where ranges of values are provided, they are also intended to include each value within the range as if set forth individually, unless expressly stated to the contrary. The use of any and all examples, or exemplary language ("e.g.," "such as," or the like) provided herein, is intended merely to better describe the embodiments and does not pose a limitation on the scope of the embodiments. No language in the specification should be construed as indicating any unclaimed element as essential to the practice of the embodiments.

In the following description, it is understood that terms such as "first," "second," "top," "bottom," "up," "down," "above," "below," and the like, are words of convenience and are not to be construed as limiting terms unless specifically stated to the contrary.

Exemplary embodiments provide physiological measurement systems, devices and methods for continuous health and fitness monitoring, and provide improvements to overcome the drawbacks of conventional heart rate monitors. One aspect of the present disclosure is directed to providing a lightweight wearable system with a strap that collects various physiological data or signals from a wearer. The strap may be used to position the system on an appendage or extremity of a user, for example, wrist, ankle, and the like. Exemplary systems are wearable and enable real-time and continuous monitoring of heart rate without the need for a chest strap or other bulky equipment which could otherwise cause discomfort and prevent continuous wearing and use. The system may determine the user's heart rate without the use of electrocardiography and without the need for a chest strap. Exemplary systems can thereby be used in not only assessing general well-being but also in continuous monitoring of fitness. Exemplary systems also enable monitoring of one or more physiological parameters in addition to heart rate including, but not limited to, body temperature, heart rate variability, motion, sleep, stress, fitness level, recovery level, effect of a workout routine on health and fitness, caloric expenditure, blood pressure, and the like.

A health or fitness monitor that includes bulky components may hinder continuous wear. Existing fitness monitors often include the functionality of a watch, thereby making the health or fitness monitor quite bulky and inconvenient for continuous wear. Accordingly, one aspect is directed to providing a wearable health or fitness system that does not include bulky components, thereby making the bracelet slimmer, unobtrusive, and appropriate for continuous wear. The ability to continuously wear the bracelet further allows continuous collection of physiological data, as well as continuous and more reliable health or fitness monitoring. For example, embodiments of the bracelet disclosed herein allow users to monitor data at all times, not just during a fitness session. In some embodiments, the wearable system may or may not include a display screen for displaying heart rate and other information. In other embodiments, the wearable system may include one or more light emitting diodes (LEDs) to provide feedback to a user and display heart rate selectively. In some embodiments, the wearable system may include a removable or releasable modular head that may provide additional features and may display additional information. Such a modular head can be releasably installed on the wearable system when additional information display is desired and removed to improve the comfort and appearance of the wearable system. In other embodiments, the head may be integrally formed in the wearable system.

Exemplary embodiments also include methods for measuring tightness of a wearable monitor and providing actionable feedback to a user. The tightness of the wearable monitor may have an impact on its performance. To help ensure a good fit, a physical model such as a spring model or resonance model may be created to characterize movement of the wearable monitor when elastically retained in tension about a body part. The wearable monitor may then be vibrated, and a response to these vibrations may be applied to the model to infer the tension. The inferred tension may be used to provide adjustment information to the user.

The term "continuous," as used herein in connection with heart rate data collection, refers to collection of heart rate data at a sufficient frequency to enable detection of individual heartbeats, and also refers to collection of heart rate data continuously throughout the day and night. More generally with respect to physiological signals that might be monitored by a wearable device, "continuous" or "continuously" will be understood to mean continuously at a rate suitable for intended time-based processing, and physically at a rate possible by the monitoring hardware, subject to ordinary data acquisition limitations such as sampling limitations and sampling rates associated with converting physical signals into digital data, and physical limitations associated with physical disruptions during use, e.g., temporary displacement of monitoring hardware due to sudden movements, changes in external lighting, loss of electrical power, physical manipulation or adjustment by a wearer, physical displacement of monitoring hardware due to external forces, and so forth. It will also be noted that heart rate data or a monitored heart rate, in this context, may more generally refer to raw sensor data, heart rate data, signal peak data, heart rate variability data, or any other physiological or digital signal suitable for recovering heart rate data as contemplated herein, and that heart rate data may generally be captured over some historical period that can be subsequently correlated to various metrics such as sleep states, activity recognition, resting heart rate, maximum heart rate, and so forth.

The term "pointing device," as used herein, refers to any suitable input interface, specifically, a human interface device, that allows a user to input spatial data to a computing system or device. In an exemplary embodiment, the pointing device may allow a user to provide input to the computer using physical gestures, for example, pointing, clicking, dragging, and dropping. Exemplary pointing devices may include, but are not limited to, a mouse, a touchpad, a touchscreen, and the like.

The term "computer-readable medium," as used herein, refers to a non-transitory storage hardware, non-transitory storage device or non-transitory computer system memory that may be accessed by a controller, a microcontroller, a computational system or a module of a computational system to encode thereon computer-executable instructions or software programs. The "computer-readable medium" may be accessed by a computational system or a module of a computational system to retrieve and/or execute the computer-executable instructions or software programs encoded on the medium. The non-transitory computer-readable media may include, but are not limited to, one or more types of hardware memory, non-transitory tangible media (for example, one or more magnetic storage disks, one or more optical disks, one or more USB flash drives), computer system memory or random access memory (such as, DRAM, SRAM, EDO RAM) and the like.

The term "distal," as used herein, refers to a portion, end or component of a physiological measurement system that is farthest from a user's body when worn by the user.

The term "proximal," as used herein, refers to a portion, end or component of a physiological measurement system that is closest to a user's body when worn by the user.

The term "equal," as used herein, refers, in a broad lay sense, to exact equality or approximate equality within some tolerance.

Exemplary embodiments provide wearable physiological measurements systems that are configured to provide continuous measurement of physiological data such as heart rate or other physiological data such as blood pressure, hydration state, blood oxygenation state, etc. Exemplary systems are configured to be continuously wearable on an appendage, for example, wrist or ankle, and do not rely on electrocardiography or chest straps in detection of heart rate. The exemplary system includes one or more light emitters for emitting light at one or more desired frequencies toward the user's skin, and one or more light detectors for received light reflected from the user's skin. The light detectors may include a photoresistor, a phototransistor, a photodiode, and the like. As light from the light emitters (for example, green light) pierces through the skin of the user, the blood's natural absorbance or transmittance for the light provides fluctuations in the photo-resistor readouts. These waves have the same frequency as the user's pulse since increased absorbance or transmittance occurs only when the blood flow has increased after a heartbeat. The system includes a processing module implemented in software, hardware or a combination thereof for processing the optical data received at the light detectors and continuously determining the heart rate based on the optical data. The optical data may be combined with data from one or more motion sensors, e.g., accelerometers and/or gyroscopes, to minimize or eliminate noise in the heart rate signal caused by motion or other artifacts (or with other optical data of another wavelength).

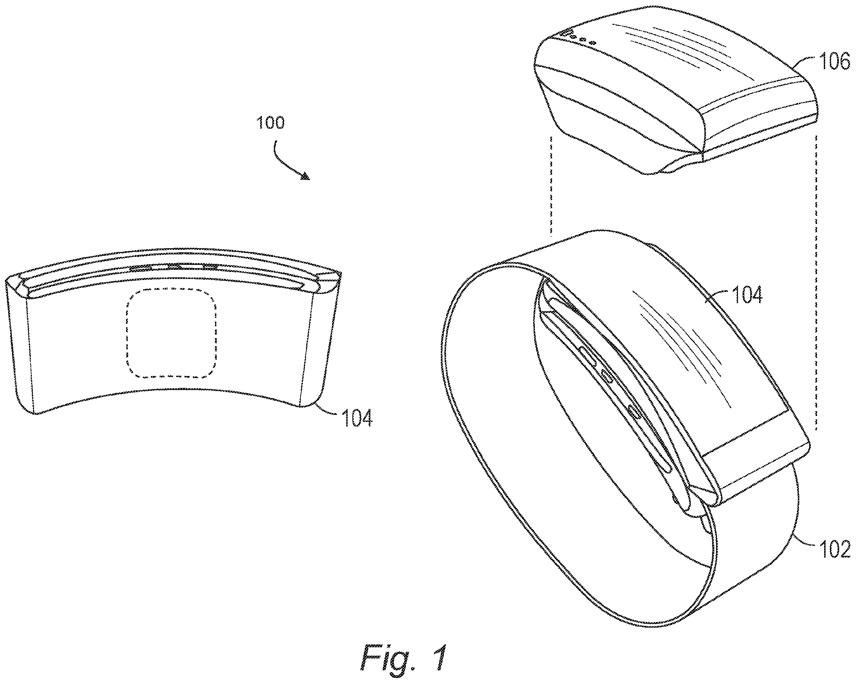

Fig. 1 shows a physiological monitoring device. The overall system 100 may include a device 104 (which may or may not include a display screen or other user interface) generally configured for physiological monitoring. The system 100 may further include a removable and replaceable battery 106 for recharging the device 104. A strap 102 may be provided, and may include any arrangement suitable for retaining the device 104 in a position on a wearer's body for acquisition of physiological data as described herein. For example, the strap 102 may include slim elastic band formed of any suitable elastic material, for example, a rubber, a woven polymer fiber such as a woven polyester, polypropylene, nylon, spandex, and so forth. The strap 102 may be adjustable to accommodate different wrist sizes, and may include any latches, hasps, or the like to secure the device 104 in an intended position for monitoring a physiological signal. While a wrist-worn device is depicted, it will be understood that the device 104 may be configured for positioning in any suitable location on a user's body, based on the sensing modality and the nature of the signal to be acquired. For example, the device 104 may be configured for use on a wrist, an ankle, a bicep, a chest, or any other suitable location(s), and the strap 102 may be, or may include, a waistband or other elastic band or the like within an article of clothing or accessory. The device 104 may also or instead be structurally configured for placement on or within a garment, e.g., permanently or in a removable and replaceable manner. To that end, the device 104 may be structurally configured for placement within a pocket, slot, and/or other housing that is coupled to or embedded within a garment. In such configurations, the garment may include sensing windows or other pathways such that the device 104 can sense physiological and/or biomechanical parameters from a user wearing a garment that includes the device 104 therein or thereon.

The system 100 may include any hardware components, subsystems, and the like to provide various functions such as data collection, processing, display, and communications with external resources. For example, the system 100 may include a heart rate monitor using, e.g., photoplethysmography, electrocardiography, or any other technique(s). The system 100 may be configured such that, when placed for use about a wrist, the system 100 initiates acquisition of physiological data from the wearer. In some embodiments, the pulse or heart rate may be taken using an optical sensor coupled with one or more light emitting diodes (LEDs), all directly in contact with the user's wrist. The LEDs may be positioned to direct illumination toward the user's skin, and may be accompanied by one or more photodiodes or other photodetectors suitable for measuring illumination from the LEDs that is reflected and/or transmitted by the wearer's skin.

The system 100 may be configured to record other physiological and/or biomechanical parameters including, but not limited to, skin temperature (using a thermometer), galvanic skin response (using a galvanic skin response sensor), motion (using one or more multi-axes accelerometers and/or gyroscope), blood pressure, and the like, as well environmental or contextual parameters such as ambient light, ambient temperature, humidity, time of day, and the like. The system 100 may also include other sensors such as accelerometers and/or gyroscopes for motion detection, and sensors for environmental temperature sensing, electrodermal activity (EDA) sensing, galvanic skin response (GSR) sensing, and the like.

The system 100 may include one or more sources of battery life, such as a first battery environmentally sealed within the device 104 and a battery 106 that is removable and replaceable to recharge the battery in the device 104. The system 100 may perform numerous functions related to continuous monitoring, such as automatically detecting when the user is asleep, awake, exercising, and so forth, and such detections may be performed locally at the device 104 or at a remote service coupled in a communicating relationship with the device 104 and receiving data therefrom. In general, the system 100 may support continuous, independent monitoring of a physiological signal such as a heart rate, and acquired data may be stored on the device 104 until it can be uploaded to a remote processing resource for more computationally expensive analysis.

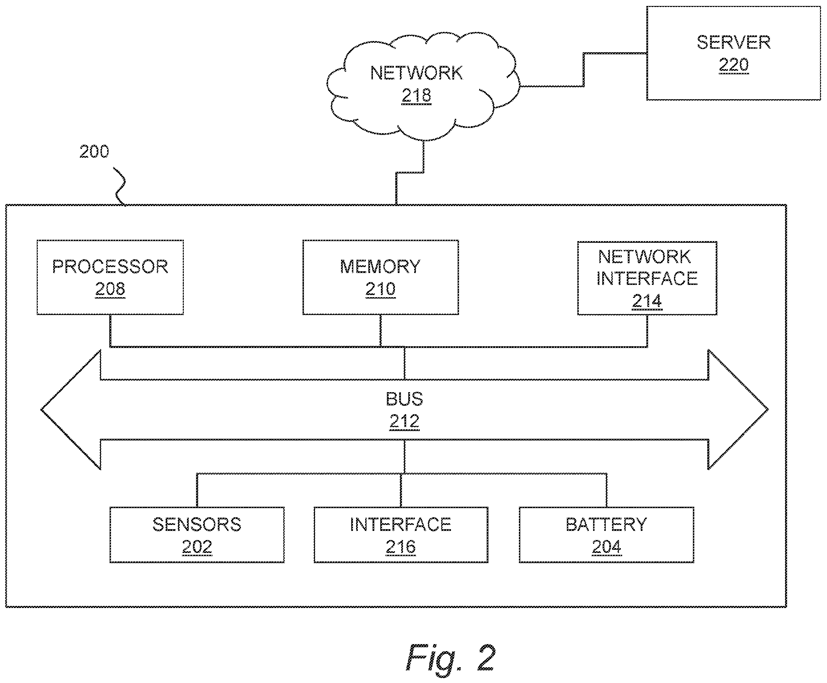

Fig. 2 is a block diagram of an exemplary computing device 200 that may be used in to perform any of the methods provided by exemplary embodiments. The computing device may, for example, be a device used for continuous physiological monitoring. The device may also or instead be any of the local computing devices described herein, such as a desktop computer, laptop computer, smart phone, and the like. The device may also or instead be any of the remote computing resources described herein, such as a web server, a cloud database, a file server, an application server, or any other remote resource or the like. While described as a physical device, it will be understood that the exemplary computing device 200 may also or instead be realized as a virtual computing device such as a virtual machine executing a web server or other remote resource in a cloud computing platform. In general, the computing device 200 may include one or more sensors 202, a battery 204, storage 206, a processor 208, a memory 210, a network interface 214, and a user interface 216, or virtual instances of one or more of the foregoing.

The sensors 202 may include any sensor or combination of sensors suitable for heart rate monitoring as contemplated herein, as well as sensors 202 for detecting calorie burn, position (e.g., through a Global Positioning System or the like), motion, activity, and so forth. In one aspect, this may include optical sensing systems including LEDs or other light sources, along with photodiodes or other light sensors, that can be used in combination for photoplethysmography measurements of heart rate, pulse oximetry measurements, and other physiological monitoring.

The sensors 202 may also or instead include one or more sensors for activity measurement. In some embodiments, the system may include one or more multi-axes accelerometers and/or gyroscope to provide a measurement of activity. In some embodiments, the accelerometer may further be used to filter a signal from the optical sensor for measuring heart rate and to provide a more accurate measurement of the heart rate. In some embodiments, the wearable system may include a multi-axis accelerometer to measure motion and calculate distance. Motion sensors may be used, for example, to classify or categorize activity, such as walking, running, performing another sport, standing, sitting, or lying down. The sensors 202 may, for example, include a thermometer for monitoring the user's body or skin temperature. In one embodiment, the sensors 202 may be used to recognize sleep based on a temperature drop, Galvanic Skin Response data, lack of movement or activity according to data collected by the accelerometer, reduced heart rate as measured by the heart rate monitor, and so forth. The body temperature, in conjunction with heart rate monitoring and motion, may be used, e.g., to interpret whether a user is sleeping or just resting, as well as how well an individual is sleeping. The body temperature, motion, and other sensed data may also be used to determine whether the user is exercising, and to categorize and/or analyze activities as described in greater detail below. In another aspect, the sensors 202 may include one or more contact sensors, such as a capacitive touch sensor or resistive touch sensor, for detecting placement of a physiological monitor for use on a user. More generally, the sensors 202 may include any sensor or combination of sensors suitable for monitoring geographic location, physiological state, exertion, movement, and so forth in any manner useful for physiological monitoring as contemplated herein.

The battery 204 may include one or more batteries configured to allow continuous wear and usage of the wearable system. In one embodiment, the wearable system may include two or more batteries, such as a removable battery that may be removed and recharged using a charger, along with an integral battery that maintains operation of the device 200 while the main battery charges. In another aspect, the battery 204 may include a wireless rechargeable battery that can be recharged using a short range or long range wireless recharging system.

The processor 208 may include any microprocessor, microcontroller, signal processor, or other processor or combination of processors and other processing circuitry suitable for performing the processing steps described herein. In general, the processor 208 may be configured by computer executable code stored in the memory 210 to provide activity recognition and other physiological monitoring functions described herein.

In general the memory 210 may include one or more non-transitory computer-readable media for storing one or more computer-executable instructions or software for implementing exemplary embodiments. The non-transitory computer-readable media may include, but are not limited to, one or more types of hardware memory, non-transitory tangible media (for example, one or more magnetic storage disks, optical disks, USB flash drives), and the like. In one aspect, the memory 210 may include a computer system memory or random access memory, such as DRAM, SRAM, EDO RAM, and the like. The memory 210 may include other types of memory as well, or combinations thereof, as well as virtual instances of memory, e.g., where the device is a virtual device. In general, the memory 210 may store computer readable and computer-executable instructions or software for implementing methods and systems described herein. The memory 210 may also or instead store physiological data, user data, or other data useful for operation of a physiological monitor or other device described herein, such as data collected by sensors 202 during operation of the device 200.

The network interface 214 may be configured to wirelessly communicate data to a server 220, e.g., through an external network 218 such as any public network, private network, or other data network described herein, or any combination of the foregoing including, e.g., local area networks, the Internet, cellular data networks, and so forth. Where the device is a physiological monitoring device, the network interface 214 may be used, e.g., to transmit raw or processed sensor data stored on the device 200 to the server 220, as well as to receive updates, receive configuration information, and otherwise communicate with remote resources and the user to support operation of the device. More generally, the network interface 214 may include any interface configured to connect with one or more networks, for example, a Local Area Network (LAN), a Wide Area Network (WAN), the Internet, or a cellular data network through a variety of connections including, but not limited to, standard telephone lines, LAN or WAN links (for example, 202.11, T1, T3, 56kb, X.25), broadband connections (for example, ISDN, Frame Relay, ATM), wireless connections, or some combination of any or all of the above. The network interface 212 may include a built-in network adapter, network interface card, PCMCIA network card, card bus network adapter, wireless network adapter, USB network adapter, modem or any other device suitable for interfacing the computing device 200 to any type of network capable of communication and performing the operations described herein. The network interface 214 may also or instead be configured to transmit and/or receive wireless signals such as Bluetooth wireless signals and the like.

The user interface 216 may include any components suitable for supporting interaction with a user. This may, for example include a keypad, display, buzzer, speaker, light emitting diodes, and any other components for receiving input from, or providing output to, a user. In one aspect, the user interface 216 may include an alarm such as a speaker (and an associated audio source), a buzzer, a haptic output, or the like configured to notify a user of an event, or to wake a user from a sleep event. In one aspect, the device 200 may be configured to receive tactile input, such as by responding to sequences of taps on a surface of the device to change operating states, display information and so forth. The user interface 216 may also or instead include a graphical user interface rendered on a display for graphical user interaction with programs executing on the processor 208 and other content rendered by a physical display of device 200.

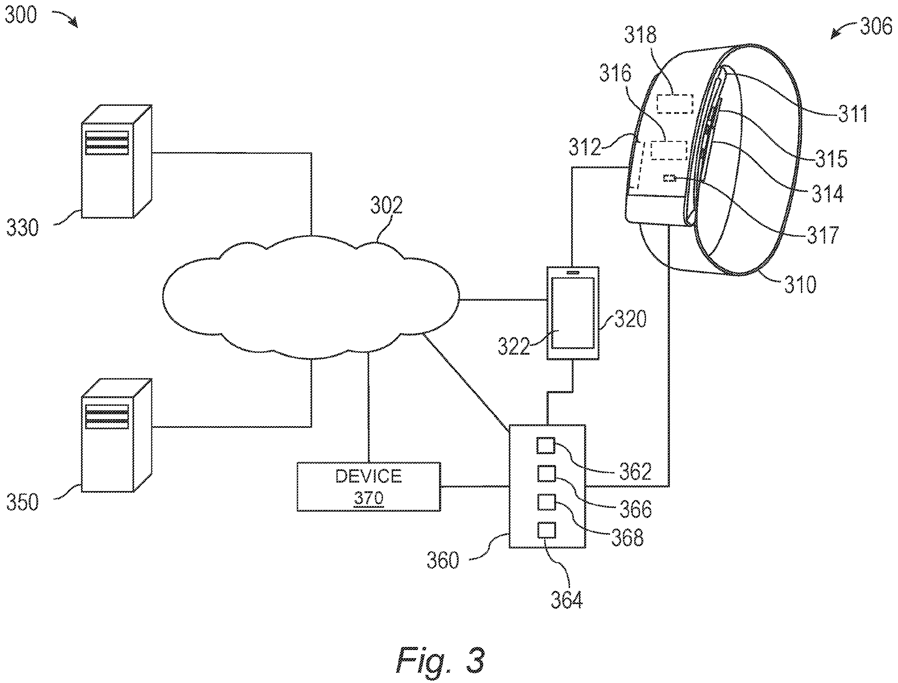

Fig. 3 illustrates an environmental and physiological monitoring system. More specifically, Fig. 3 illustrates a system 300 facilitating environmental and physiological monitoring that may be used with any of the methods or devices described herein. In general, the system 300 may include a physiological monitor 306, a user device 320, a remote server 330 with a remote data processing resource (such as any of the processors or processing resources described herein), an environmental monitor 360, a device 370 for interaction with an environment, and one or more other resources 350, all of which may be interconnected through a data network 302.

The data network 302 may be any of the data networks described herein. For example, the data network 302 may be any network(s) or internetwork(s) suitable for communicating data and information among participants in the system 300. This may include public networks such as the Internet, private networks, telecommunications networks such as the Public Switched Telephone Network or cellular networks using third generation (e.g., 3G or IMT-2000), fourth generation (e.g., LTE (E-UTRA) or WiMAX-Advanced (IEEE 802.16m)), fifth generation (e.g., 5G), and/or other technologies, as well as any of a variety of corporate area or local area networks and other switches, routers, hubs, gateways, and the like that might be used to carry data among participants in the system 300. This may also include local or short range communications networks suitable, e.g., for coupling the physiological monitor 306 to the user device 320 and/or environmental monitor 360, or otherwise communicating with local resources.

The physiological monitor 306 may, in general, be any physiological monitoring device, such as any of the wearable monitors or other monitoring devices described herein. Thus, the physiological monitor 306 may generally be shaped and sized to be worn on a wrist or other body location and retained in a desired orientation relative to the appendage with a strap 310 or other attachment mechanism. The physiological monitor 306 may include a wearable housing 311, a network interface 312, one or more sensors 314, one or more light sources 315, a processor 316, a memory 318, an output device 317 such as a haptic device and/or any other type of component suitable for providing haptic or other sensory alerts to a user, and a wearable strap 310 for retaining the physiological monitor 306 in a desired location on a user. In one aspect, the output device 317 may include an alarm, e.g., for providing a notification to a user of an event, or for waking a user from a sleep event. It will be understood that, while such an alarm is depicted as being a component of the monitor 306, an alarm may also or instead be included in one of the other devices described herein such as the user device 320, the environmental monitor 360, or the device 370.

In general, the physiological monitor 306 may include a wearable physiological monitor configured to acquire heart rate data and/or other physiological data from a wearer. More specifically, the wearable housing 311 of the physiological monitor 306 may be configured such that a user can wear a wearable physiological monitor 306 configured to acquire heart rate data and/or other physiological data from the user in a substantially continuous manner. The wearable housing 311 may be configured for cooperation with a strap 310 or the like, e.g., for engagement with an appendage of a user. The wearable housing 311 may also or instead be configured for placement on or within a garment to be worn by a user.

The network interface 312 may be configured to coupled one or more participants of the system 300 in a communicating relationship, e.g., with the remote server 330, either directly, e.g., through a cellular data connection or the like, or indirectly through a short range wireless communications channel coupling the physiological monitor 306 locally to a wireless access point, router, computer, laptop, tablet, cellular phone, or other device that can relay data from the physiological monitor 306 to the remote server 330 as necessary or helpful for acquiring and processing data.

The one or more sensors 314 may include any of the sensors described herein, or any other sensors suitable for physiological monitoring. By way of example and not limitation, the one or more sensors 314 may include one or more of a light source, and optical sensor, an accelerometer, a gyroscope, a temperature sensor, a galvanic skin response sensor, a capacitive sensor, a resistive sensor, an environmental sensor (e.g., for measuring ambient temperature, humidity, lighting, and the like), a geolocation sensor, a temporal sensor, an electrodermal activity sensor, and the like. The one or more sensors 314 may be disposed in the wearable housing 311, or otherwise positioned and configured for capture of data for physiological monitoring of a user. In one aspect, the one or more sensors 314 include a light detector configured to provide data to the processor 316 for calculating a heart rate variability. The one or more sensors 314 may also or instead include an accelerometer configured to provide data to the processor 316, e.g., for detecting activities such as a sleep state, a waking event, exercise, and/or other user activity. In an implementation, the one or more sensors 314 measure a galvanic skin response of the user.

The processor 316 and memory 318 may be any of the processors and memories described herein, and may be suitable for deployment in a physiological monitoring device. In one aspect, the memory 318 may store physiological data obtained by monitoring a user with the one or more sensors 314. The processor 316 may be configured to obtain heart rate data from the user based on the data from the sensors 314. The processor 316 may be further configured to assist in a determination of a condition of the user, such as whether the user has an infection or other condition of interest as described herein.

The one or more light sources 315 may be coupled to the wearable housing 311 and controlled by the processor 316. At least one of the light sources 315 may be directed toward the skin of a user's appendage. Light from the light source 315 may be detected by the one or more sensors 314.

The system 300 may further include a remote data processing resource executing on a remote server 330. The remote data processing resource may be any of the processors described herein, and may be configured to receive data transmitted from the memory 318 of the physiological monitor 306, and to process the data to detect or infer physiological signals of interest such as heart rate, heart rate variability, respiratory rate, pulse oxygen, blood pressure, body temperature, skin temperature, and so forth. The remote server 330 may also or instead evaluate a condition of the user such as a recovery state, sleep quality, daily activity strain, and any health conditions that might be detected based on such data.

The system 300 may also include one or more user devices 320, which may work together with the physiological monitor 306 and/or the environmental monitor 360, e.g., to provide a display for user data and analysis, and/or to provide a communications bridge from the network interface 312 of the physiological monitor 306 and/or the environmental monitor 360 to the data network 302 and the remote server 330. For example, one or more of the physiological monitor 306 and the environmental monitor 360 may communicate locally with each other and/or a user device 320, such as a smartphone of a user, via short-range communications, e.g., Bluetooth, or the like, e.g., for the exchange of data between the physiological monitor 306, the environmental monitor 360, and the user device 320, where the user device 320 may communicate with the remote server 330 via the data network 302. Computationally intensive processing may be performed at the remote server 330, which may have greater memory capabilities and processing power than the physiological monitor 306 that acquires the data. However, it will be understood that processing may also or instead be performed at one or more of the physiological monitor 306, the environmental monitor 360, the user device 320, the device 370, and so on. That is, it will be understood that one or more of the steps related to techniques for environmental monitoring and control as described herein, or sub-steps, calculations, functions, and the like related thereto, can be performed locally, remotely, or some combination of these. Thus, steps may be performed locally on a wearable device and/or environmental monitor 360, remotely on a server or other remote resource, on an intermediate device such as a local computer used by the user to access the remote resource, or any combination of these. For example, using the example system 300 of Fig. 3, one or more steps of a technique for environmental monitoring and control may, wholly or partially, be performed locally on one or more of the physiological monitor 306, the environmental monitor 360, and the user device 320, such as by training a machine learning model to detect deviations from a typical sleep pattern, and then pruning or otherwise optimizing the machine learning model for deployment on the wearable device. Also, or instead, one or more steps of a technique for environmental monitoring and control may, wholly or partially, be performed remotely on one or more of the remote server 330 and the other resource(s) 350. Thus, for example, where a wearable monitor and an environmental monitor 360 are positioned sufficiently near a smartphone of the user for short range wireless communications during sleep, heart rate data and environmental data may be continuously or periodically transmitted to the remote server 330, which may monitor received data to detect disturbances from sleep caused by an environmental condition. Other combinations are also possible.

The user device 320 may include any computing device as described herein, including without limitation a smartphone, a desktop computer, a laptop computer, a network computer, a tablet, a mobile device, a portable digital assistant, a cellular phone, a portable media or entertainment device, and so on. The user device 320 may provide a user interface 322 for access to data and analysis by a user, and/or to control operation of one or more of the physiological monitor 306, the environmental monitor 360, and the device 370. The user interface 322 may be maintained by a locally-executing application on the user device 320, or the user interface 322 may be remotely served and presented on the user device 320, e.g., from the remote server 330 or the one or more other resources 350.

In general, the remote server 330 may include data storage, a network interface, and/or other processing circuitry. The remote server 330 may process data from one or more of the physiological monitor 306 and the environmental monitor 360, and the remote server 330 may perform any of the analyses described herein, and may host a user interface for remote access to this data, e.g., from the user device 320. The remote server 330 may include a web server or other programmatic front end that facilitates web-based access by the user devices 320, the physiological monitor 306, and/or the environmental monitor 360 to the capabilities of the remote server 330 or other components of the system 300.

The other resources 350 may include any resources that can be usefully employed in the devices, systems, and methods as described herein. For example, these other resources 350 may include without limitation other data networks, human actors (e.g., programmers, researchers, annotators, editors, analysts, and so forth), sensors (e.g., audio or visual sensors), data mining tools, computational tools, data monitoring tools, algorithms, and so forth. The other resources 350 may also or instead include any other software or hardware resources that may be usefully employed in the networked applications as contemplated herein. For example, the other resources 350 may include payment processing servers or platforms used to authorize payment for access, content, or option/feature purchases, or otherwise. In another aspect, the other resources 350 may include certificate servers or other security resources for third-party verification of identity, encryption or decryption of data, and so forth. In another aspect, the other resources 350 may include a desktop computer or the like co-located (e.g., on the same local area network with, or directly coupled to through a serial or USB cable) with a user device 320, wearable strap 310, environmental monitor 360, and/or remote server 330. In this case, the other resources 350 may provide supplemental functions for components of the system 300.

The other resources 350 may also or instead include one or more web servers that provide web-based access to and from any of the other participants in the system 300. While depicted as a separate network entity, it will be readily appreciated that the other resources 350 (e.g., a web server) may also or instead be logically and/or physically associated with one of the other devices described herein, and may for example, include or provide a user interface 322 for web access to a remote server 330 or a database in a manner that permits user interaction through the data network 302, e.g., from the physiological monitor 306, the environmental monitor 360, and/or the user device 320.

The environmental monitor 360 may include one or more sensors 364 configured to monitor conditions in an environment in which the environmental monitor 360 is placed. The environmental monitor 360 may also or instead be configured to communicate with the physiological monitor 306 and/or other participants in the system 300, e.g., in order to provide recommendations related to an environment, and/or to control an environment, for the benefit of a wearer of the physiological monitor 306. In this manner, and similar to the physiological monitor 306, the environmental monitor 360 may include a network interface 362, a processor 366, a memory 368, and so on, where one or more of these components may be the same or similar to any as described herein. Further functionality and example use cases for the environmental monitor 360 within a system such as the system 300 of Fig. 3 are described below.

The device 370 may be structurally configured for interaction with an environment. By way of example, the device 370 may include one or more of a light fixture, a light bulb, an entertainment device (e.g., a television, a radio, and so on), a portion of an HVAC system (e.g., a thermostat), a portion of a window or the like (e.g., a curtain, a shade, and so on), a sound machine or the like, a household appliance, and so on. In this manner, the device 370 may be a "smart" device, controllable via a computing device or the like such as one or more of the user device 320, the physiological monitor 306, the environmental monitor 360, and so on. By way of example, one use case for the system 300 may be to improve the sleep of a wearer of the physiological monitor 306 using data obtained from one or more of the physiological monitor 306, the environmental monitor 360, and the device 370. Continuing with this example, the physiological monitor 306 may detect that a user is awakened or disturbed at a certain time of night on a certain day of the week, and the environmental monitor 360 may similarly detect a relatively loud noise that occurs during that same time of night and day of the week (e.g., from garbage collection or the like). And, furthering this example, in order to remediate this disturbance, the device 370 may be activated-e.g., the device 370 may include a sound machine that is activated during the night, before the time of the disturbance, in order to drown out the noise from the disturbance, or the device 370 may include electronically activated noise blocking curtains that can be drawn closed during the night before the time of the disturbance, or similar. Other examples and use cases are also or instead possible, where some are described below.

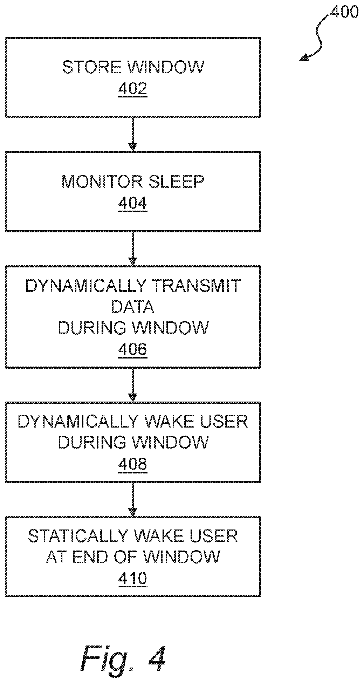

Fig. 4 is a flow chart of a method for providing a configurable wake up alarm. In general, an alarm system as described herein may monitor physiological data for a user, and use the physiological data to determine when during a user-configured window to provide a wake up signal to the user. The system may advantageously use low rate, low resolution, and/or low frequency data communications before an onset of the user-configured window in order to conserve battery life for a battery powered physiological monitor that is acquiring the physiological data, and/or to conserve processing resources at a remote server that might, for example, perform relatively computationally expensive calculations to evaluate sleep state, sleep quality, and the like in order to determine an optimal wake up time.

As shown in step 402, the method 400 may include storing a window-e.g., a window configured by a user and/or a computer for waking the user from a sleep event. The window may generally specify an interval of time (also referred to herein as a "waking interval") that is bounded by an onset of a waking interval and an end of the waking interval. This window facilitates a variable wake time that may occur at a time between the onset and the end (inclusive), depending on factors such as the duration and quality of sleep preceding the issue of an alarm, a user's sleep need, and so forth. It will be understood that the window may be stored on a wearable device, e.g., in a memory of a wearable physiological monitor or other wireless monitoring device such as any of those described herein. Also or instead, the window may be configured and subsequently stored on another computing device, such as a local or remote device associated with the user or otherwise associated with the wearable device. The window may also be communicated among any of the devices described herein, e.g., as necessary or helpful for selecting a time to issue a wake up alarm.

The wearable device may, for example, include any of the wearable devices described herein, such as a wearable photoplethysmography device or any other monitoring device for acquiring heart rate data or other physiological data associated with sleep events and/or sleep quality for a user. The wearable device may, in general, include a haptic output, an audio output, a visual output, or any other output suitable for waking a user at a designated time. In another aspect, e.g., where the user's environment includes smart home devices and the like, these smart home devices may be controlled alone or in combination to wake the user, e.g., by playing sounds over a home audio system, by changing room temperature, by increasing powered lighting, by opening shades, and so forth. In another aspect, outputs such as audio from a personal electronic device (e.g., smart phone, laptop, etc.) may be controlled to similarly provide a waking stimulus for the user.

The wearable device may include a wireless interface such as a Bluetooth interface, WiFi interface, or other proprietary or standard wireless short-range communications interface for wirelessly sending and receiving data. This interface may be used, e.g., to communicate or receive information concerning the window, to receive a wake signal, to communicate the wake signal to nearby output devices, to transmit physiological data to a remote processing resource, e.g., at the onset of the window, and so forth. In one aspect, a user interface for configuring the window may be provided by a smart phone, tablet, laptop computer, desktop computer, and/or other device associated with the user.

The window may include an end of a waking interval such as a hard stop provided by the user at which time the user must wake up. For example, if a user must wake at a certain time to attend a class, catch an airplane flight, attend a meeting, go to work, make an appointment, etc., the desired or intended wake time may be entered as an end to the wake window. The user may also or instead specify an onset of a waking interval, e.g., the start of the window, based on an earliest time at which the user is willing to wake up. In another aspect, the onset and/or end of the window may be automatically calculated for the user. For example, the onset to the window may be automatically calculated in response to a selection by the user of the end of the window and may be based on a naive window assumption (e.g., one hour before the end of the window) or knowledge about the user's sleep habits and history, which permits a calculation of the earliest likely time at which the user will satisfy the user's sleep need, or a user-specified portion of the sleep need such as 90% of sleep need. In another aspect, the end of the window may be automatically calculated in response to a selection by the user of the onset of the window. That is, a user may specify a target amount of sleep and an end to the window may be selected in response.

The window may be further configurable, e.g., automatically and/or manually via the user interface or the like. By way of example, a system may include controls for a user to create a recurring alarm schedule from the user interface on the user's computing device This recurring alarm schedule (and associated calculated windows) may be stored on the wearable physiological monitor or another component of the system (e.g., on a local computing device and/or a backend server or database), and may be used on a recurring basis to generate alarms for the user. The schedule for the recurring alarm schedule may be controlled by the user, e.g., by specifying days of the week (e.g., Monday through Friday), specifying recurring dates (e.g., the first Thursday of every month, or April 1st each year), or specifying particular dates, such as by selecting dates on a calendar for use of the recurring alarm. Other possible configurations of the alarm are also or instead possible, including for example, a daily alarm schedule, a weekly alarm schedule, a monthly alarm schedule, and so forth. The alarm schedule for certain events may include certain associated settings such as variations to a minimum amount of sleep, a minimum quality of sleep or sleep score, a latest wake time, and so forth. This may be useful for event-based alarm schedules such as a holiday alarm schedule, a workday alarm schedule, a vacation alarm schedule, a training and/or exercise alarm schedule, an activity schedule (e.g., games/matches for an athlete), and so forth. And, in some aspects, an alarm schedule (and associated calculated windows) may be coordinated-automatically and/or manually-with a physiological and/or hormonal cycle of the user, such as a menstrual cycle and the like.

In another aspect, the onset and/or end of the window may be automatically selected for the user. For example, the onset and/or the end of the window may be determined based on a calculated sleep need for the user. This may be based on a sleep history for the user, a prior day strain for the user, a prior day recovery of the user, or some combination of these. In general, the actual quality and quantity of sleep can be monitored as described herein to determine whether to issue an intermittent wake signal to the user between the onset and end of the window.

As shown in step 404, the method 400 may include monitoring sleep of the user, e.g., by acquiring physiological data with the monitoring device and analyzing the physiological data using any suitable processing techniques. This may include acquiring heart rate data, e.g., with a wearable physiological monitor and/or photoplethysmography device, or any other physiological monitor or combination of monitors suitable for monitoring a physiological signal associated with sleep quality. Useful techniques for detecting sleep, categorizing sleep types, detecting waking events or sleep interruptions, evaluating sleep duration and quality, and scoring sleep are described by way of non-limiting examples in U.S. Pat. No. 9,743,848 issued on August 29, 2017 and entitled "HEART RATE VARIABILITY WITH SLEEP DETECTION," U.S. Pat. No. 10,182,726 issued on January 22, 2019 and entitled "DEVICE FOR PHYSIOLOGICAL MONITORING AND MEASUREMENT," U.S. Pat. Pub. No. 2022/0183618 filed on December 10, 2020 and entitled "DETECTING SLEEP INTENTION," and U.S. Pat. Pub. No. 2022/0273269 filed on March 24, 2021. These techniques may be used to monitor and evaluate sleep as described herein.

It will be understood that monitoring sleep of the user may also or instead include monitoring sleep with any of the environmental monitoring systems and devices described herein. Data concerning temperature, background noise, changes in lighting, and the like may be useful in evaluating the quality of sleep by a user. This environmental data may be retrieved by a computing resource that will evaluate for possible waking times in order to refine an estimate of the amount of sleep a user might need or desire. Similarly, data such as motion within a room where a user is sleeping (and or motion measured by the wearable device worn by the user) may also or instead be used to refine an evaluation of the quality of sleep enjoyed by a user during a sleep session.

As shown in step 406, the method 400 may include dynamically transmitting data such as the acquired physiological data from the monitoring device to a remote processing system. For example, this may include, at the onset of the window, increasing a frequency of communication of the physiological data from the wireless monitoring device to the remote processing system, e.g., so that the remote processing system can process the data to evaluate a current sleep state of the user based on an available history of related physiological data. In one aspect, the increase in frequency may be a binary change from a low frequency, low resolution, and/or low data rate communication to a higher frequency, resolution, data type, and/or data rate so that more complete physiological data can be communicated for use by the remote processing system to evaluate prior sleep duration and quality, as well as a current sleep state. In another aspect, the increase in frequency may be a batch transfer of data acquired during a preceding sleep session. The higher data rate may be sustained throughout the window so that the sleep state of the user can be relatively frequently updated by the remote processing system in order to identify a suitable waking moment. In another aspect, the data rate or data frequency may vary throughout the window based on, e.g., a current estimated sleep need, battery reserves for the monitoring device, and/or any other constraints or parameters.

As shown in step 408, the method 400 may include dynamically waking the user within the window. For example, if the user has a sufficient amount and quality of sleep (based on historical analysis of the user, or based on explicit user inputs or sleep requirements), the remote processing system may determine that it is appropriate to awaken the user by issuing a wake signal to the monitoring device (or other system suitable for issuing a wake alarm to the user) before the end of the window. By way of further example, based on historical analysis of the user, a certain sleep quality metric or sleep need may be determined for a user for any given night. This metric may be customized for the user, and may vary over time based on a user's activity, diet, sleep, or otherwise. The user may review this metric as an aid for manually configuring when the user would like to be awakened within the window-e.g., by setting a wake up for when the user reaches 100% of their sleep need, when the user reaches 90% of their sleep need, when the user reaches 75% of their sleep need, and so on. As another example, once high frequency data is being transmitted, the recipient (e.g., a server or the like) may identify where the user is in a current sleep cycle to see if a natural waking point can be predicted within the window. If a natural waking moment or waking interval is identified, e.g., at or near the end of a period of Rapid Eye Movement (REM) sleep or at the onset of a following light sleep cycle, the alarm may be configured to wake the user at that moment or interval. More generally, the remote processing system may evaluate sleep of the user based on the physiological data and determine whether to issue the wake signal based on the quality of sleep, the duration of sleep, the stage of sleep, or some combination of these. Using these or other techniques, the remote processing system may identify a suitable waking time during the window, and may transmit an output to trigger an alarm. A suitable waking time may also or instead be locally estimated where suitable processing resources are locally available, e.g., on the monitoring device or a local computing device for the user.

If this wake signal is received from the remote processing system during the window, the recipient device may generate an output in order to wake the user at a wake time within the window specified by the wake signal. The output may include, e.g., a haptic signal on a wearable device, an audible beep or alert from the wearable device or another device associated with the user, or the like. In one aspect, the wake signal may include an explicit timestamp indicating when to issue a corresponding output, or the wake signal may provide an instruction to a recipient device to issue a waking output that can be processed substantially upon receipt by a corresponding alarm output system. As noted above, while the foregoing generally contemplates the use of remote resources to analyze sleep and/or calculate a suitable waking time, this processing may also be performed locally, e.g., on a user's local computing device, or, if the physiological monitor has sufficient processing resources, directly on the physiological monitor. Even in these instances, e.g., where it is not necessary to transmit data to a remote server for processing, the local device(s) may benefit from selective use of processing resources to conserve battery power, avoid processing during times of low activity, and so forth.

It will also be appreciated that the remote processing system may include a remote server, a personal computing device for the user, or some combination of these. Thus, for example, a suitably programmed and suitably powerful personal computer, laptop, smart phone, or other personal computing device or the like nearby the user may process physiological data locally and evaluate the quality of sleep to determine whether it is appropriate to wake a user before the end of the window. In another aspect, a personal computing device may provide an intermediate communications system for transferring physiological data from the monitoring device to a remote server accessible through a data network, cellular network, or some combination of these. The personal computing device may also or instead transfer wake signals, automatically calculated window parameters such as the onset or the end of the window, and so forth to the monitoring device and/or other supporting devices such as a user smart phone, a smart home system, and so forth.

As shown in step 410, the method 400 may include statically waking the user at the end of the window, e.g., as distinguished from dynamically and/or conditionally waking the user based on a waking signal from a remote resource prior to the end of the window. If no wake signal is received during the window, the monitoring device (or other alarm output system) will default to issuing an alarm at the end of the window. In general, this ensures that the user does not sleep past a maximum desired waking time. This also provides a backstop to ensure that an output is generated to wake the user even in the absence of network connectivity, short range wireless connectivity, server failure, personal computing device shutdown, or any other event or combination of events that might interfere with sleep evaluation and response by a remote resource.

According to the foregoing, a system described herein includes a wearable physiological monitoring device worn by a user (e.g., the physiological monitor 306 shown in Fig. 3), a personal electronic device associated with the user and coupled to the wearable physiological monitoring device through a wireless interface (e.g., the user device 320 shown in Fig. 3), and a remote processing resource coupled through a data network to the personal electronic device (e.g., the remote server 330 coupled through the data network 302 to the user device 320 as shown in Fig. 3). The wearable physiological monitoring device may include a memory (e.g., the memory 318 in Fig. 3) storing a window timewise bounded by an onset and an end configured by a user for waking the user from a sleep event, as well as a haptic output device (e.g., the haptic device 317 of Fig. 3) and a first wireless interface (e.g., the network interface 312 of Fig. 3). The personal electronic device may include an interface for the user to configure the window (e.g., the user interface 322 shown in Fig. 3) and a second wireless interface coupled in a communicating relationship with the first wireless interface of the wearable physiological monitoring device. The remote processing resource may be configured by computer executable code embodied in a non-transitory computer readable medium to receive physiological data acquire by the wearable physiological monitoring device and communicated to the remote processing resource through the personal electronic device, to analyze the physiological data, and to conditionally issue a wake signal to the wearable physiological monitoring device prior to the end of the window when an analysis of the physiological data shows an optimum time to wake the user before the end of the window. The wearable physiological monitor may be responsive to a receipt of the wake signal from the remote processing resource by outputting a signal to the haptic output device to wake the user.

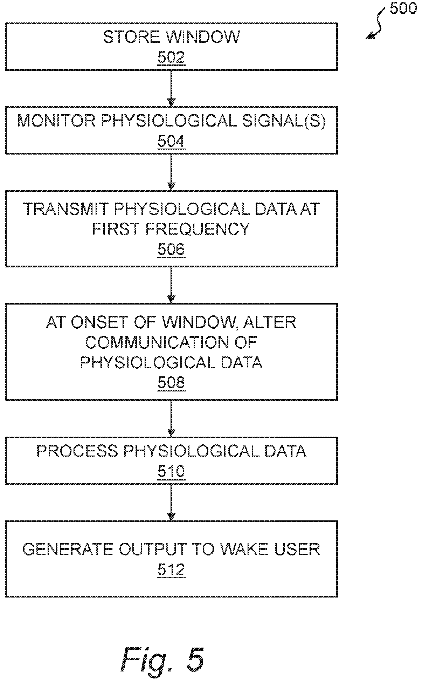

Fig. 5 is a flow chart of a method for generating a waking alarm. The method 500 may be performed using any of the devices and systems described herein.

As shown in step 502, the method 400 may include storing a window such as any of the windows described herein-e.g., a window configured by a user for waking the user from a sleep event-on a wearable monitor. The window may generally specify an interval of time that is timewise bounded by an onset of a waking interval and an end of the waking interval. This window facilitates a variable wake time that may occur at any time as early as the onset of a waking interval and as late as the end of the waking interval, depending on factors such as the duration and quality of sleep preceding the issue of an alarm, a user's sleep need, and so forth. It will be understood that the window may be stored on a wearable device, e.g., in a memory of a wearable physiological monitor or other wireless monitoring device such as any of those described herein. Also or instead, the window may be configured and subsequently stored on another computing device, such as a local or remote device associated with the user or otherwise associated with the wearable device. The window may also be communicated among any of the devices described herein, e.g., as necessary or helpful for managing issuance of the wake up alarm.

In some aspects, the user provides an end to the waking interval defined by the window as a time when the user must wake up. Similarly, in some aspects, the user provides the onset of the waking interval as an earliest time when the user might wish to wake up. Additionally or alternatively, at least one of the onset of the window and the end of the window may be automatically calculated for the user. For example, the onset of the window may be automatically calculated in response to a selection by the user of the end of the window. Also or instead, the end of the window may be automatically calculated in response to a selection by the user of the onset of the window. In some aspects, at least one of the onset and the end of the window may be calculated for the user based on a sleep need of the user, a prior day strain for the user, and/or the like.

As stated above, the wearable device may be any of the devices described herein. This may include, for example, a wrist-worn physiological monitor, a physiological monitor worn on another appendage or other part of the body of a user, a physiological monitor engaged with and/or embedded within a garment, a photoplethysmography device, and the like.

As shown in step 504, the method 500 may include monitoring a physiological signal, e.g., a physiological signal associated with sleep quality, sleep phase, and/or sleep duration, with a physiological monitor to acquire physiological data. For example, this may include monitoring a heart rate signal with a wearable heart rate monitor to acquire heart rate data. In some aspects, this data is used at least in part to generate a wake signal. For example, the remote processing system may evaluate sleep of the user based on the physiological data, and may determine whether to issue the wake signal based on a quality of the sleep, a duration of the sleep, a completion of a number of sleep cycles, or some combination of these. Also or instead, the remote processing system may evaluate sleep of the user based on the physiological data, and may determine whether to issue the wake signal based on a current stage of the sleep, or more generally, may control the wake signal to occur to occur at a predetermined stage of sleep, such as entering light sleep after a REM sleep stage. In another aspect, environmental data may be analyzed and used to estimate a quality of sleep by the user, in order to refine or otherwise adjust the calculation of a suitable time for issuing a wake signal within the wake interval.

The remote processing system may be any as described herein. For example, the remote processing system may include a smart phone associated with the user and the wireless monitoring device. Also or instead, the remote processing system may include a remote server configured to analyze sleep performance based on the physiological signal, identify a suitable wake time within the waking interval, and/or transmit a wake signal to an output device, or otherwise support a dynamic, configurable wake up alarm as described herein.

As shown in step 506, the method 500 may include periodically transmitting physiological data through a user device (e.g., a smart phone) to a remote processing system during sleep at a first frequency. The first frequency may be set such that these periodic transmissions conserve a battery of the physiological monitor, and/or conserve the processing resources of a system that receives the transmissions and performs computationally complex data processing. Thus, the first frequency may be lower in content or rate, than a frequency used to transmit data when a user is active and/or awake.

As shown in step 508, the method 500 may include, at the onset of the window, altering an attribute related to communication of the physiological data from the monitoring device to the remote processing system. For example, this may include transmitting the physiological data to the remote processing system at a second frequency greater than the first frequency described above. This may also or instead include transmitting greater detail or otherwise increasing information communicated to the remote resource to assist in evaluation of a current sleep status. More generally, when an onset of the waking interval is reached, indicating that a waking time or event for the user may be approaching, the frequency of the transmission of physiological data, or the amount or resolution of transmitted data, may be increased relative to when a user is (presumably) sleeping before the onset of the window. Thus, it will be understood that, in some aspects, altering the attribute related to communications includes increasing of one or more of a frequency, a resolution, and a data rate of the physiological data.

As shown in step 510, the method 500 may include processing the physiological data at the remote processing system to determine whether a wake signal should be issued from the remote processing system to awaken the user during the waking interval, calculate a suitable wake time within the wake interval, and/or transmit a wake signal to an output device to facilitate delivery of a wake up alarm at an appropriate time.

As shown in step 512, the method 500 may include generating an output to wake the user. For example, this may include, if the wake signal is received from the remote processing system during the window, generating an output to wake the user at a wake time within the window specified by the wake signal. Specifically, this may include generating a haptic output with the physiological monitor to wake the user at a wake time within the window specified by the wake signal, or otherwise generating an audio output, mechanical output, or other output or control signal suitable for waking the user from sleep. In one aspect, the wake signal may include a timestamp indicating a wake up time calculated by the remote processing system at which an output is to be delivered. In another aspect, the wake signal may omit the timestamp, e.g., where the wake signal is intended for immediate execution by the wearable device (or other output device) to generate a user alert. In one aspect, if the wake signal is not received from the remote processing system during the window, generating the output may include generating the output to wake the user at the end of the window. Specifically, this may include generating a haptic output or other stimulus to wake the user at the end of the window.

In general, the output may be generated by a haptic device and/or an audio device on the wireless monitoring device, and may include any suitable buzzing, audio alert, or the like. Also or instead, the output may be generated by an audio and/or visual component on a smart phone associated with the user, or another user device with audio and/or visual capabilities (e.g., a smart alarm, a smart television, personal digital device, an Internet of Things (IoT) device, a sound machine, a home automation device, and so on). In some aspects, the wake signal may be transmitted to a controller of a smart home product, such as window shades, lights, thermostats, or the like that may be controlled (either together or alone) to generate a waking stimulus for the user.

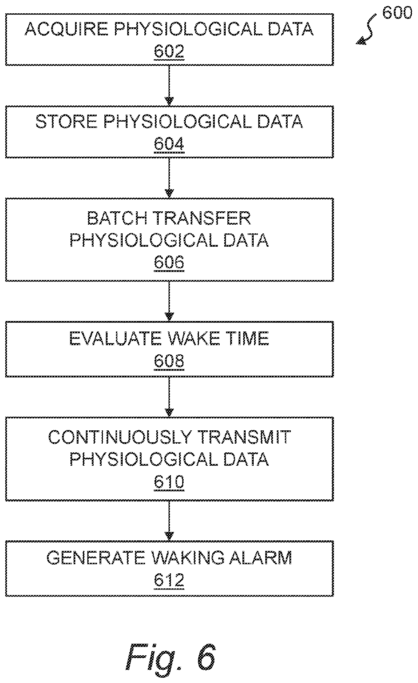

Fig. 6 is a flow chart of a method for generating a waking alarm. The method 600 may be performed using any of the devices and systems described herein.

As shown in step 602, the method 600 may include acquiring physiological data from a user, e.g., with a wearable device such as any of those described herein.

As shown in step 604, the method 600 may include storing the physiological data, e.g., in a memory of the wearable device.

As shown in step 606, the method 600 may include batch transferring the physiological data to a remote processing resource at a beginning of a window for waking the user, such as the automatically or manually established onset of a waking interval. This may include any of the physiological data stored in the memory of the wearable device, such as data logged locally during sleep but not yet transmitted to the remote processing resource. The remote processing resource may be a server or other computing device, such as any of those described herein, configured to evaluating a wake time for the user based on an analysis of the physiological data.

As shown in step 608, the method 600 may include evaluating, by the remote processing resource or another computing resource connected thereto, a wake time for the user at a beginning of a window for waking the user. This may include calculating or estimating a suitable wake time based on the physiological data that was batch-transferred at the beginning of the window, such as data accumulated on the physiological monitor during a preceding period of sleep.

As shown in step 610, the method 600 may include continuously transmitting additional physiological data to the remote processing resource during the window. In general, once the waking interval has begun, data may be streamed continuously in order to update evaluation of a possible waking signal based on additional data concerning a current sleep state, real time sleep interruptions, possible waking opportunities, and so forth. In another aspect, the remote processing resource may perform a single evaluation of a possible waking time based on the initial batch transfer of data received at the onset of the waking interval, and determine a suitable waking time (if any) based on the initial batch transfer.

As shown in step 612, the method 600 may include generating a waking alarm for the user at an earliest of (1) an expiration of the window, e.g., the end of the waking interval, or (2) a wake time received from the remote processing resource, e.g., upon receipt of a wake signal from the remote processing resource or at a time specified by the wake signal received from the remote processing resource. The waking alarm may use any of the output devices and techniques described herein.

The above systems, devices, methods, processes, and the like may be realized in hardware, software, or any combination of these suitable for the control, data acquisition, and data processing described herein. This includes realization in one or more microprocessors, microcontrollers, embedded microcontrollers, programmable digital signal processors or other programmable devices or processing circuitry, along with internal and/or external memory. This may also, or instead, include one or more application specific integrated circuits, programmable gate arrays, programmable array logic components, or any other device or devices that may be configured to process electronic signals. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software.

Thus, in one aspect, each method described above, and combinations thereof may be embodied in computer executable code that, when executing on one or more computing devices, performs the steps thereof. In another aspect, the methods may be embodied in systems that perform the steps thereof, and may be distributed across devices in a number of ways, or all of the functionality may be integrated into a dedicated, standalone device or other hardware. The code may be stored in a non-transitory fashion in a computer memory, which may be a memory from which the program executes (such as random access memory associated with a processor), or a storage device such as a disk drive, flash memory or any other optical, electromagnetic, magnetic, infrared or other device or combination of devices. In another aspect, any of the systems and methods described above may be embodied in any suitable transmission or propagation medium carrying computer-executable code and/or any inputs or outputs from same. In another aspect, means for performing the steps associated with the processes described above may include any of the hardware and/or software described above. All such permutations and combinations are intended to fall within the scope of the present disclosure.

The method steps of the implementations described herein are intended to include any suitable method of causing such method steps to be performed, consistent with the patentability of the following claims, unless a different meaning is expressly provided or otherwise clear from the context. So, for example, performing the step of X includes any suitable method for causing another party such as a remote user, a remote processing resource (e.g., a server or cloud computer) or a machine to perform the step of X. Similarly, performing steps X, Y, and Z may include any method of directing or controlling any combination of such other individuals or resources to perform steps X, Y, and Z to obtain the benefit of such steps. Thus, method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity and need not be located within a particular jurisdiction.

It will be appreciated that the methods and systems described above are set forth by way of example and not of limitation. Numerous variations, additions, omissions, and other modifications will be apparent to one of ordinary skill in the art. In addition, the order or presentation of method steps in the description and drawings above is not intended to require this order of performing the recited steps unless a particular order is expressly required or otherwise clear from the context. Thus, while particular embodiments have been shown and described, it will be apparent to those skilled in the art that various changes and modifications in form and details may be made therein without departing from the scope of this disclosure and are intended to form a part of the invention as defined by the following claims.

## Claims

1. A method comprising:
storing a window on a wearable heart rate monitor, the window configured by a user for waking the user from a sleep event and the window timewise bounded by an onset of a waking interval and an end of the waking interval;
monitoring a heart rate signal associated with sleep quality with the wearable heart rate monitor to acquire heart rate data;
periodically transmitting the heart rate data through a smart phone of the user to a remote processing resource during the sleep event at a first frequency;
at the onset of the window, transmitting the heart rate data to the remote processing resource at a second frequency greater than the first frequency, wherein the remote processing resource is configured to analyze sleep performance for the user based on the heart rate data and to determine whether a wake signal should be issued from the remote server processing resource to awaken the user before the end of the window based on a sleep need of the user;
if a wake signal is received from the remote processing system during the window, generating a haptic output with the wearable heart rate monitor to wake the user at a wake time within the window specified by the wake signal; and
if the wake signal is not received from the remote processing system during the window, generating the haptic output to wake the user at the end of the window.

2. The method of claim 1, further comprising, at the onset of the window, altering an attribute related to communication of the heart rate data to the remote processing resource, the attribute including at least one of a resolution, and a data rate of the heart rate data.

3. The method of any of claims 1 to 2, wherein the user provides the end to the window as a time when the user must wake up.

4. The method of any of claims 1 to 3, wherein the user provides the onset to the window as an earliest time when the user wishes to wake up.

5. The method of any of claims 1 to 3, wherein the onset of the window is automatically calculated in response to a selection by the user of the end of the window.

6. The method of any of claims 1 to 2 or claim 4, wherein the end of the window is automatically calculated in response to a selection by the user of the onset of the window.

7. The method of any of claims 1 to 6, wherein at least one of the onset and the end of the window is automatically calculated for the user.

8. The method of any of claims 1 to 7, wherein at least one of the onset and the end of the window is calculated for the user based on a sleep need of the user.

9. The method of any of claims 1 to 8, wherein at least one of the onset and the end of the window is calculated for the user based on a prior day strain for the user.

10. The method of any of claims 1 to 9, wherein the remote processing resource evaluates sleep of the user based on the heart rate data and determines whether to issue the wake signal based on a quality of the sleep.

11. The method of any of claims 1 to 10, wherein the remote processing resource evaluates sleep of the user based on the heart rate data and determines whether to issue the wake signal based on a stage of the sleep.

12. The method of any of claims 1 to 11, wherein the wearable heart rate monitor includes a wrist-worn photoplethysmography device.

13. The method of any of claims 1 to 12, wherein the remote processing resource includes at least one of (a) a smart phone associated with the user and the wearable heart rate monitor and (b) a remote server.

14. The method of any of claims 1 to 13, wherein the wake signal includes a timestamp indicating a wake up time calculated by the remote processing resource.

15. The method of any of claims 1 to 14 wherein the remote processing resource is configured to issue the wake signal when the user reaches at least one of 75% of sleep need, 90% of sleep need, or 100% of sleep need.

## Patentansprüche

1. Verfahren, umfassend:
Speichern eines Fensters auf einem tragbaren Herzfrequenzmonitor, wobei das Fenster von einem Benutzer so ausgebildet wird, dass es den Benutzer aus einem Schlafereignis weckt, und wobei das Fenster zeitlich durch den Beginn eines Wachintervalls und das Ende des Wachintervalls begrenzt ist;
Überwachen eines mit der Schlafqualität assoziierten Herzfrequenzsignals mit dem tragbaren Herzfrequenzmonitor, um Herzfrequenzdaten zu erfassen;
periodisches Übertragen der Herzfrequenzdaten über ein Smartphone des Benutzers an eine entfernte Verarbeitungsressource während des Schlafereignisses mit einer ersten Frequenz;
zu Beginn des Fensters, Übertragen der Herzfrequenzdaten an die entfernte Verarbeitungsressource mit einer zweiten Frequenz, die höher ist als die erste Frequenz, wobei die entfernte Verarbeitungsressource ausgebildet ist, um die Schlafleistungsfähigkeit des Benutzers basierend auf den Herzfrequenzdaten zu analysieren und basierend auf einem des Schlafbedarf des Benutzers zu bestimmen, ob von der entfernten Server-Verarbeitungsressource ein Wecksignal ausgegeben werden soll, um den Benutzer vor dem Ende des Fensters zu wecken;
falls während des Fensters ein Wecksignal vom entfernten Verarbeitungssystem empfangen wird, Erzeugen einer haptischen Ausgabe mit dem tragbaren Herzfrequenzmonitor, um den Benutzer zu einem Weckzeitpunkt innerhalb des Fensters, spezifiziert durch das Wecksignal, zu wecken; und
falls während des Fensters kein Wecksignal vom entfernten Verarbeitungssystem empfangen wird, Erzeugen der haptischen Ausgabe, um den Benutzer am Ende des Fensters zu wecken.

2. Verfahren nach Anspruch 1, weiter umfassend, zu Beginn des Fensters, Ändern eines Attributs, das die Kommunikation der Herzfrequenzdaten an die entfernte Verarbeitungsressource betrifft, wobei das Attribut mindestens eines aus einer Auflösung und einer Datenrate der Herzfrequenzdaten einschließt.

3. Verfahren nach einem der Ansprüche 1 bis 2, wobei der Benutzer das Ende des Fensters als einen Zeitpunkt bereitstellt, zu dem er aufwachen muss.

4. Verfahren nach einem der Ansprüche 1 bis 3, wobei der Benutzer den Beginn des Fensters als einen frühesten Zeitpunkt bereitstellt, zu dem er aufwachen möchte.

5. Verfahren nach einem der Ansprüche 1 bis 3, wobei der Beginn des Fensters automatisch als Antwort auf eine Auswahl des Endes des Fensters durch den Benutzer berechnet wird.

6. Verfahren nach einem der Ansprüche 1 bis 2 oder Anspruch 4, wobei das Ende des Fensters automatisch als Antwort auf eine Auswahl des Beginns des Fensters durch den Benutzer berechnet wird.

7. Verfahren nach einem der Ansprüche 1 bis 6, wobei mindestens eines aus dem Beginn oder dem Ende des Fensters automatisch für den Benutzer berechnet wird.

8. Verfahren nach einem der Ansprüche 1 bis 7, wobei mindestens eines aus dem Beginn oder dem Ende des Fensters für den Benutzer basierend auf einem Schlafbedarf des Benutzers berechnet wird.

9. Verfahren nach einem der Ansprüche 1 bis 8, wobei mindestens eines aus dem Beginn oder dem Ende des Fensters für den Benutzer basierend auf einer Belastung am Vortag für den Benutzer berechnet wird.

10. Verfahren nach einem der Ansprüche 1 bis 9, wobei die entfernte Verarbeitungsressource den Schlaf des Benutzers basierend auf den Herzfrequenzdaten bewertet und basierend auf einer Schlafqualität bestimmt, ob das Wecksignal ausgegeben werden soll.

11. Verfahren nach einem der Ansprüche 1 bis 10, wobei die entfernte Verarbeitungsressource den Schlaf des Benutzers basierend auf den Herzfrequenzdaten bewertet und basierend auf einer Schlafphase bestimmt, ob das Wecksignal ausgegeben werden soll.

12. Verfahren nach einem der Ansprüche 1 bis 11, wobei der tragbare Herzfrequenzmonitor eine am Handgelenk getragene Photoplethysmographie-Vorrichtung einschließt.

13. Verfahren nach einem der Ansprüche 1 bis 12, wobei die entfernte Verarbeitungsressource mindestens eines aus (a) einem Smartphone, das mit dem Benutzer und dem tragbaren Herzfrequenzmonitor assoziiert ist, und (b) einem entfernten Server einschließt.

14. Verfahren nach einem der Ansprüche 1 bis 13, wobei das Wecksignal einen Zeitstempel einschließt, der eine von der entfernten Verarbeitungsressource berechnete Weckzeit angibt.

15. Verfahren nach einem der Ansprüche 1 bis 14, wobei die entfernte Verarbeitungsressource ausgebildet ist, um das Wecksignal auszugeben, wenn der Benutzer mindestens eines aus 75 % des Schlafbedarfs, 90 % des Schlafbedarfs oder 100 % des Schlafbedarfs erreicht.

## Revendications

1. Procédé comprenant :
le stockage d'une fenêtre sur un moniteur de rythme cardiaque portable, la fenêtre étant configurée par un utilisateur pour réveiller l'utilisateur d'un événement de sommeil et la fenêtre étant délimitée temporellement par un début d'un intervalle de réveil et une fin de l'intervalle de réveil ;
la surveillance d'un signal de rythme cardiaque associé à une qualité de sommeil avec le moniteur de rythme cardiaque portable pour acquérir des références de rythme cardiaque ;
la transmission périodique des références de rythme cardiaque par l'intermédiaire d'un smart-phone de l'utilisateur à une ressource de traitement à distance pendant l'événement de sommeil à une première fréquence ;
au début de la fenêtre, la transmission des références de rythme cardiaque à la ressource de traitement à distance à une deuxième fréquence supérieure à la première fréquence, dans lequel la ressource de traitement à distance est configurée pour analyser des performances de sommeil de l'utilisateur sur la base des références de rythme cardiaque et pour déterminer si un signal de réveil doit être délivré par la ressource de traitement à distance de serveur pour réveiller l'utilisateur avant la fin de la fenêtre sur la base d'un besoin de sommeil de l'utilisateur ;
si un signal de réveil est reçu en provenance du système de traitement à distance pendant la fenêtre, la génération d'une sortie haptique avec le moniteur de rythme cardiaque portable pour réveiller l'utilisateur à un temps de réveil dans la fenêtre spécifié par le signal de réveil ; et
si le signal de réveil n'est pas reçu du système de traitement à distance pendant la fenêtre, la génération de la sortie haptique pour réveiller l'utilisateur à la fin de la fenêtre.

2. Procédé selon la revendication 1, comprenant en outre, au début de la fenêtre, la modification d'un attribut lié à une communication des références de rythme cardiaque à la ressource de traitement à distance, l'attribut incluant au moins l'une parmi une résolution, et un rythme de références des références de rythme cardiaque.

3. Procédé selon l'une quelconque des revendications 1 à 2, dans lequel l'utilisateur fournit la fin à la fenêtre comme un temps où l'utilisateur doit se réveiller.

4. Procédé selon l'une quelconque des revendications 1 à 3, dans lequel l'utilisateur fournit le début à la fenêtre comme un tout premier temps où l'utilisateur souhaite se réveiller.

5. Procédé selon l'une quelconque des revendications 1 à 3, dans lequel le début de la fenêtre est automatiquement calculé en réponse à une sélection par l'utilisateur de la fin de la fenêtre.

6. Procédé selon l'une quelconque des revendications 1 à 2 ou la revendication 4, dans lequel la fin de la fenêtre est automatiquement calculée en réponse à une sélection par l'utilisateur du début de la fenêtre.

7. Procédé selon l'une quelconque des revendications 1 à 6, dans lequel au moins l'un parmi le début et la fin de la fenêtre est automatiquement calculé pour l'utilisateur.

8. Procédé selon l'une quelconque des revendications 1 à 7, dans lequel au moins l'un parmi le début et la fin de la fenêtre est calculé pour l'utilisateur sur la base d'un besoin de sommeil de l'utilisateur.

9. Procédé selon l'une quelconque des revendications 1 à 8, dans lequel au moins l'un parmi le début et la fin de la fenêtre est calculé pour l'utilisateur sur la base d'une tension de la journée précédente pour l'utilisateur.

10. Procédé selon l'une quelconque des revendications 1 à 9, dans lequel la ressource de traitement à distance évalue le sommeil de l'utilisateur sur la base des références de rythme cardiaque et détermine s'il faut délivrer le signal de réveil sur la base d'une qualité du sommeil.

11. Procédé selon l'une quelconque des revendications 1 à 10, dans lequel la ressource de traitement à distance évalue le sommeil de l'utilisateur sur la base des références de rythme cardiaque et détermine s'il faut délivrer le signal de réveil sur la base d'une phase du sommeil.

12. Procédé selon l'une quelconque des revendications 1 à 11, dans lequel le moniteur de rythme cardiaque portable inclut un dispositif de photopléthysmographie porté au poignet.

13. Procédé selon l'une quelconque des revendications 1 à 12, dans lequel la ressource de traitement à distance inclut au moins l'un parmi (a) un smart-phone associé à l'utilisateur et au moniteur de rythme cardiaque portable et (b) un serveur à distance.

14. Procédé selon l'une quelconque des revendications 1 à 13, dans lequel le signal de réveil inclut un horodatage indiquant un temps de réveil calculé par la ressource de traitement à distance.

15. Procédé selon l'une quelconque des revendications 1 à 14, dans lequel la ressource de traitement à distance est configurée pour délivrer le signal de réveil lorsque l'utilisateur atteint au moins une valeur parmi 75 % de besoin de sommeil, 90 % de besoin de sommeil ou 100 % de besoin de sommeil.
